# EUROPEAN PATENT SPECIFICATION

(11) **EP 2 630 487 B1**
(45) Date of publication and mention of the grant of the patent: **17.08.2016**
(21) Application number: 10768012.6
(22) Date of filing: 18.10.2010
(51) Int. Cl.: G01N 33/50, G01N 33/569, C12Q 1/06, G01N 33/58

(54) **METHOD FOR DETERMINING A DEGREE OF MASTITIS INFECTION**
VERFAHREN ZUR BESTIMMUNG EINES GRAD DER MASTITISINFEKTION
MÉTHODE POUR DÉTERMINER UN DEGRÉ DE MASTITE INFECTION

(43) Date of publication of application: 28.08.2013
(73) Proprietor: FOSS Analytical A/S, 3400 Hillerød (DK)
(72) Inventor: HOLM, Claus, DK-2100 København (DK)
(86) International application number: PCT/EP2010/065615
(87) International publication number: WO 2012/052046

(56) References cited:
- US-B1- 6 979 550
- S. HOJSGAARD AND C. FRIGGENS: "Quantifying degree of mastitis from common trends ina panel of indicators for mastitis in dairy cows.", JOURNAL OF DAIRY SCIENCE, vol. 93, no. 2, 1 February 2010 (2010-02-01), pages 582-592, XP002643464,
- GONZALO C ET AL: "Short Communication: Evaluation of the Overall Accuracy of the DeLaval Cell Counter for Somatic Cell Count in Ovine Milk: Effect of Soak Time in Diluted and Undiluted Milk Samples", JOURNAL OF DAIRY SCIENCE, AMERICAN DAIRY SCIENCE ASSOCIATION, US, vol. 91, no. 8, 1 August 2008 (2008-08-01) , pages 3114-3118, XP026954983, ISSN: 0022-0302 [retrieved on 2008-08-01]

## Description

The present invention relates to a method for determining a degree of mastitis infection from a differential somatic cell count on mammalian milk, particularly to such a method for determining the presence and/or degree of mastitis and most particularly bovine mastitis.

Mastitis is the inflammation of the mammary gland of a mammal typically caused by bacterial infection which, depending on its degree, can cause severe loss in milk yield as well as undesirable changes to the milk quality. It has been estimated that the annual economic loss for farmers in the USA alone due to bovine mastitis is over $2 billion. A substantial portion of this is estimated to be due to sub-clinical mastitis.

Clinical mastitis is typically treated with a relatively expensive course of antibiotics. Subclinical mastitis is not necessarily treated with antibiotics during lactation. Often subclinical infections are eliminated by the immune system, but subclinical mastitis may occasionally lead to clinical mastitis or remain as a subclinical infection (chronic mastitis).

Identification of mastitis, particularly bovine mastitis, is typically based on the total number of somatic cells in a predetermined volume of milk, typically using either imaging or flow cytometry to make the somatic cell count. When making this somatic cell count leukocytes (white blood cells) and epithelial (skin) cells are typically counted without any discrimination, particularly between the different types of leukocytes present in the milk, being made.

Use of the total somatic cell count as one of a panel of indicators employed to quantify the degree of mastitis infection of a cow is disclosed by Hojsgaard and Friggins, Journal of Dairy Science, vol. 93, no. 2, 1 February 2010, pages 582-592. Furthermore, total somatic cell count measurement in ovine milk is disclosed by Gonzalo et al., Journal of Dairy Science, vol. 91, no. 8, 1 August 2008, pages 3114-3118.

In the following the lymphocyte, monocyte and macrophage types of leukocyte will be referred to as 'Group A' leukocytes and polymorphonuclear or PMN (neutrophils, eosinophils and basophils) types of leukocyte will be referred to as 'Group B' leukocytes.

It is generally understood Group A members account for around 85% of the total number of somatic cells under normal conditions whereas Group B members account for approximately 10% of somatic cells under normal conditions and increase to between 30% and 90% of the total somatic cell count, dependent on the degree of mastitis.

It has been long proposed that a differential somatic cell count, being a measure of the relative proportions of Group A and Group B leukocytes and even the proportion of types of leukocytes within one or both of these groups, should be employed as an indication of one or more of the presence or degree of and/or susceptibility to mastitis.

Differential cell counting is typically achieved by means of a differentiating marker, such as a meta-chromatic stain or fluorescent or radioactive microbeads, which has one or more measurable characteristics that varies dependent on the type of cell it becomes associated with. A cytometer is then employed to count events (cells) using a detection system sensitive to the differences in these characteristics, such as changes in fluorescent properties, to differentiate between the cell types in a known manner.

It is known from, for example, US 2009/0233329, to perform a differential somatic cell count using a 'smear test' which employs a novel wedge shaped microfluidic chamber in which is placed a mixture of milk sample and a meta-chromatic stain. Here 80µl of milk is diluted with 20µl of a meta-chromatic stain and a small drop of the mixture is applied to the chamber. Identification of the different cells according to their coloration or morphology is made either manually or automatically using Image analysis. A problem with this is that a new microfluidic chamber must be used with each test. Furthermore the height of the imaging chamber may cause a blurring of the image which may lead to complications in the identification of the cells.

Flow cytometry is a well known alternative to the above described smear test type assay. In an article by M. Hageltorn and M. Alaa Saad published in Am. J. Vet. Res., pp 2012-2016, Vol. 47, No. 9, September 1986 it is disclosed that flow cytometry may be used to distinguish different leukocyte populations. Here a fresh milk sample (no more than 6 hours old) was diluted in a ratio of 1:200 with a hypotonic saline solution containing 0.0004% acridine orange meta-chromatic stain and the assay is performed using standard fluorescent flow cytometry. This dilution step is performed to effectively eliminate the possibility of a coincidence of the cells to be measured and interfering particles (such as fat particles) at the measurement point but adds complexity to the assay technique. Moreover the high dilution requires that a very much larger volume of liquid is assayed before a statistically significant cell count can be achieved. This in turn significantly increases the time for the assay.

The performance of differential somatic cell count using a standard flow cytometry assay technique is also described, for example, in US 6,979,550. Here it is disclosed that in order to perform the differential cell counting the somatic cells must first be isolated from interfering particles in the milk sample. This is done here by centrifuging the milk sample in order to remove interfering particles such as fat particles and concentrate the particles of interest. A fluorescent stain is added to discriminate all cells from interfering particles. Particle counting is then performed in a standard flow cytometer using an event detecting system compatible with the fluorescent stain employed. The centrifuging increases the complexity of the assay and in some cases can even modify the sample so that it is no longer representative of the whole.

It is an aim of the present invention to address at least one of the problems associated with known cytometry assay techniques for differential somatic cell counting.

Accordingly, the present invention provides a method for determining a degree of mastitis infection comprising the steps of i) preparing an un-isolated sample by adding a reagent containing a differentiating marker, such as meta-chromatic stain, fluorescent or radioactive microspheres, in an amount sufficient to provide a differentiation between cell types to mammalian milk to dilute it in a range less than 1:200, preferably less than 1:50 and most preferably less than 1:5; ii) measuring a differential somatic cell count on the sample by means of a flow cytometer having a detection system sensitive to differences in the differentiating marker resulting from the marker becoming differently associated with different cell types in the sample; and iii) determining an indication of a degree of infection dependent on the measured differential cell count.

Thus, the inventor has surprisingly discovered that by judicious choice of the amount of differentiating marker added to a sample a cytometry assay is able to be performed on an un-isolated, essentially undiluted, sample in order to make a differential somatic cell count measurement by monitoring the differentiating characteristic(s) of the marker itself, the results of which may be used to determine an indication of the degree of mastitis infection. The deviation of these differentiating characteristic(s) from a norm and/or the extent of deviation may be used to identify the existence of, stage of and/or likelihood of contracting mastitis.

Usefully the indication of the degree infection may include the identification of one or more of the presence, likelihood or level of mastitis. Such identification may be employed in a number of herd management decisions such as the milking order according to mastitis status, better flushing of milking claw after milking an infected cow or using a separate milking claw for infected cows, in order to lower cow-to-cow infection risk; grouping of cows with subclinical mastitis, in order to lower cow-to-cow infection risk; predicting if a subclinical mastitis infection will evolve into a clinical case and consequently identifying those cows likely to benefit from antibiotic treatment; and culling and breading selections.

The present invention will now be illustrated by way of non-limiting examples and with reference to the drawings of the accompanying figures of which:
Fig. 1 shows a dot plot of side scatter intensity vs green fluorescence by flow cytometry in a milk sample in which cells are marked according to the present invention;
Fig. 2 shows a dot plot of red vs green fluorescence of the gated region illustrated in Fig.1;
Fig. 3 shows a dot plot of red vs green fluorescence of the gated region illustrated in Fig.2 in which different cell populations are identified;
Fig. 4 shows a graph of %PMN measured on Day 0 (fresh milk) vs %PMN measured on subsequent days using a method according to the present invention;
Fig. 5 shows a typical ungated dot plot of red vs green fluorescence by flow cytometry in a milk sample prepared in accordance with the values of Table 2;
Fig. 6 shows a dot plot of red vs green fluorescence from a milk sample stained with a relatively low concentration of acridine orange;
Fig. 7 shows a dot plot of red vs green fluorescence illustrating the effect of adding EDTA to a sample stained with a concentration as employed in Fig. 5;
Fig. 8 shows a dot plot of red vs green before PBS is added; and
Fig. 9 shows the effects of adding PBS.

### EXAMPLE 1

An amount of 50µl cold and preserved (with BSM II tabs) milk from an individual cow is transferred into an Eppendorf tube. 50µl acridine orange in PBS (1mg/ml) and 10µl Na₂H₂EDTA (0.1 g/ml, pH=7) are added one reagent at a time and mixed. Furthermore, 100µl PBS is then added to the above mention mixture and mixed. After reacting for 1-2 minutes at room temperature, the mixture was measured on a standard flow cytometer. In this flow cytometer, and by way of example only, the sample is flowed past the detection region with a speed of 14µl/min. As the differentiating marker is a meta-chromatic stain cell types can be differentiated using cell fluorescence. The stain employed in the present example is acridine orange and so fluorescence is, in the present example, excited by a blue laser (488nm) with the intensities of green (here designated FL1-H) and red (here designated FL2-H) being monitored. A FL1-H value of 400,000 is used as trigger signal. In the present arrangement light scatter signals are also acquired and the intensity of the side scatter light (here designated SSC-H) is advantageously employed to reduce noise (i.e. signals not related to the cells to be counted) from the acquired signal.

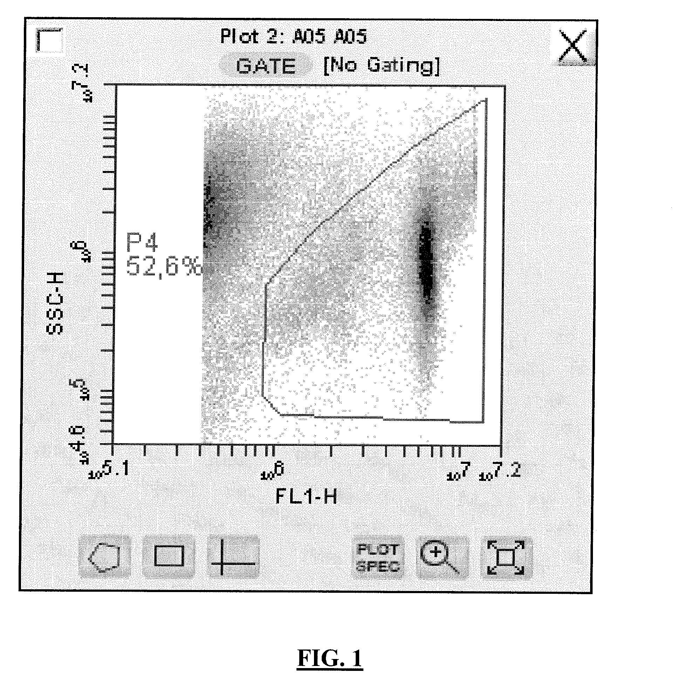

For data analysis, the side scatter vs. green fluorescence is plotted and is shown in Fig. 1. In this plot a gate is drawn to define where in the plot the events of interest, in this example cells, are located. Particles showing significant side scatter (SSC-H) signals but relatively low green fluorescence (FL1-H) signals may be readily identified as uninteresting and eliminated from further analysis. The exact parameters for the gate may be readily determined by the skilled person using standard techniques known in the art of flow cytometry.

Thus noise particles are eliminated or at least significantly reduced. This gated region is identified as P4 and in the present example represents 52.6% of the total number of counted events.

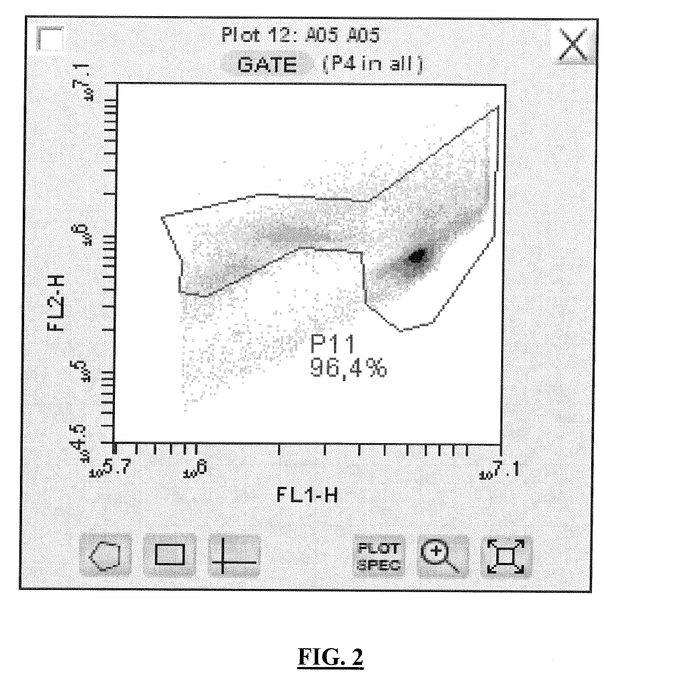

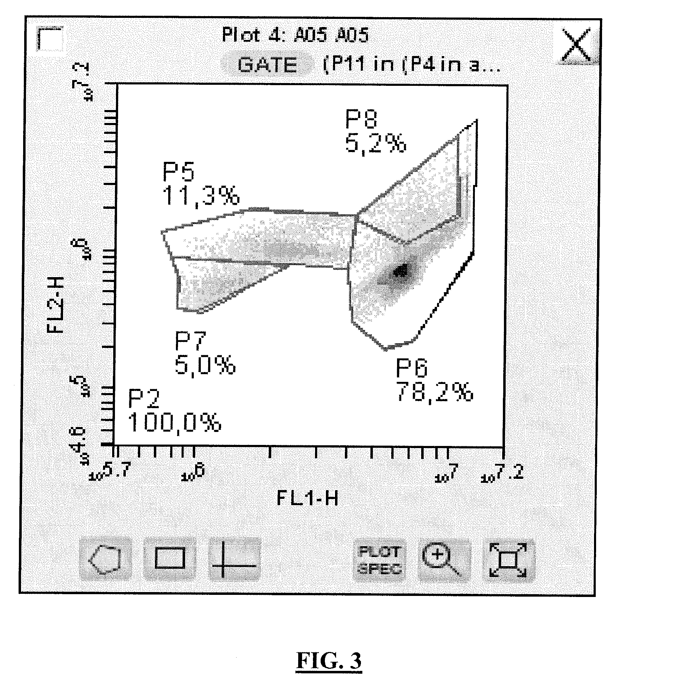

The gated events P4 are then plotted in a red (FL2-H) vs. green (FL1-H) fluorescence plot as illustrated in Fig.2. Usefully, but not essentially, in this plot a second gate is drawn to define where in the plot the events of interest (cells) are located and thus additional noise particles are eliminated. This gated region is identified as P11 and in the present example represents 96.4% of the total number of counted events in the P4 gated region and corresponds to a total somatic cell count of 450,000 cells per millilitre (cells/ml). This plot may be employed in the determination of the percentage of cells belonging to Groups A and B however preferably the gate defining all cells in the P11 region of Fig. 2 is then subdivided into regions defining different cell populations based on the relative ratios of fluorescence detected at wavelengths characteristic of the meta-chromatic stain employed, here acridine orange with associated red/green (FL2-H/FL1-H) fluorescence intensities. The P11 region from Fig. 2 is in the present Fig.3 identified as P2. The regions of different cell types are illustrated as P5, P6, P7 and P8 in Fig. 3 and from visual observation are considered to most likely be comprised of monocytes (Group A); PMN (Group B); lymphocytes (Group A) and macrophages (Group A) respectively. Once these visual correlations have been made then the ratios of characteristic fluorescence associated with these regions may be employed in future analysis without relying on the step of visual identification.

The number of events in the region P6 which defines the so called Group B (or PMN) cell region in Fig.3 is divided with the total number of cells and multiplied with 100 to obtain the %Group B (PMN) which, in the present example, is 78.2%.

In order to establish an indication of the degree of infection from this result a comparison may be made with reference values established using milk from cows with a known degree of infection. This comparison may be made mathematically using conventional regression or chemometric techniques applied to the reference values in order to establish a mathematical relationship between cell count and degree of infection. The result from an unknown sample may then be processed in a data processor using the previously established mathematically relationship in order to arrive at an indication of the degree of infection of the unknown cow. Alternatively, ranges of cell counts may be indexed against mastitis diagnosis and the measured cell counts is then compared to the ranges in order to provide an indication of a degree of infection. This is illustrated by way of example only in Table 1 below and may be done either manually or automatically by means of a data processor.

**Table 1**

| Somatic cell count (cells/ml) | %PMN | Mastitis diagnosis |
|---|---|---|
| >500,000 | - | Clinical or subclinical mastitis |
| 400,001 - 500,000 | ≥30 | Subclinical mastitis |
| 400,001 - 500,000 | <30 | No mastitis |
| 300,001 - 400,000 | ≥40 | Subclinical mastitis |
| 300,001 - 400,000 | <40 | No mastitis |
| 200,001 - 300,000 | ≥50 | Subclinical mastitis |
| 200,001 - 300,000 | <50 | No mastitis |
| 100,001 - 200,000 | ≥60 | Subclinical mastitis |
| 100,001 - 200,000 | <60 | No mastitis |
| 50,000 - 100,000 | ≥70 | Subclinical mastitis |
| 50,000 - 100,000 | <70 | No mastitis |
| <50,000 | - | No mastitis |

Thus, in the present example a PMN value of 78%, together with the total somatic cell count of 450,000 indicates subclinical mastitis. It will be appreciated by those skilled in the art that the indication of the degree of infection may be then presented to a user in a number of different ways without departing from the invention as claimed, such as YES/NO to the presence of one or both mastitis and subclinical mastistis or as a level of infection or as prediction of contracting mastitis.

### EXAMPLE 2

Milk from an individual cow is preserved (with BSM II tabs) and heated to 40°C prior to analysis. A volume of 50µl milk is transferred into an Eppendorf tube. 160µl of an acridine orange reagent (0.3mg/ml acridine orange and 0.03mg/ml Na₂H₂EDTA in PBS) is added and mixed. After reacting for one minute at room temperature, the mixture was measured on the standard flow cytometer used in Example 1. In the flow cytometer the sample is measured with a speed of 100µl/min and is again excited by a blue laser (488nm). A FL1-H value of 200,000 is used as trigger signal. Fluorescent signals (FL1-H, FL2-H and light scatter signals (side scatter, SSC-H) are again acquired.

The data analysis is similar to the one used in Example 1 the results are the same, indicating a PMN level of around 78.2%. This illustrates that the method according to the present invention is well suited to industrial (as opposed to research) application since flow speeds may be made relatively high and the additives may be pre-mixed and added as a single reagent without significant loss in predictive accuracy.

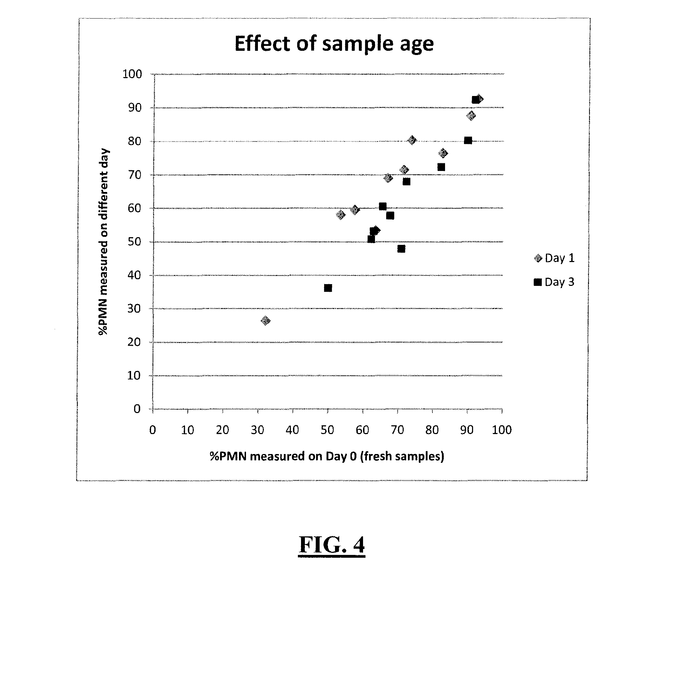

Using the same experimental parameters measurements were made on nine further samples and measurements repeated after one day (Day 1 in Fig. 4) and again after three days (Day 3 in Fig. 4). The results are illustrated in Fig. 4 and show that the Day 1 measurement results are comparable to those results obtained when the sample was fresh (Day 0 in Fig. 4) and that after three days the measured values are only slightly lower than those of Day 1 (and fresh). This indicates that the method according to the present invention may be usable to determine an indication of a degree of infection even on samples preserved for up to three days. This is an advantage when using the method to measure milk samples received at a central laboratory from dairy farmers some distances away. In this case the samples received are typically one or two day old preserved samples. Moreover, this indicates that the method may also be usable to distinguish viable cells, since mortality increases with age.

It is an essential feature of the present invention that an appropriate amount of differentiating marker, for example acridine orange meta-chromatic stain, is added to a sample which is sufficient to permit somatic cell differentiation using flow cytometry but which does not adversely dilute the sample by providing an separation effect as discussed above. An appropriate level may be determined empirically, using reasonable trial and error, by visual inspection of the appropriate dot plots as will be described below by way of example only:

The following reagents were prepared: 1mg/ml acridine orange in PBS, 0.1g/ml Na₂H₂EDTA (pH=7) and normal strength PBS. A milk sample with different types of cells are used and mixed with the reagents using the following combinations:

**Table 2**

| | | | | |
|---|---|---|---|---|
| 1 | 50µl | 50µl acridine | | 100µl PBS |
| | milk | orange | | |
| 2 | 90µl | 10µl acridine | | |
| | milk | orange | | |
| 3 | 99µl | 2µl acridine | | 100µl PBS |
| | milk | orange | | |
| 4 | 50µl | 50µl acridine | 10µl | |
| | milk | orange | EDTA | |
| 5 | 90µl | 10µl acridine | 10µl | 100µl PBS |
| | milk | orange | EDTA | |
| 6 | 99µl | 2µl acridine | 10µl | |
| | milk | orange | EDTA | |
| 7 | 50µl | 50µl acridine | | |
| | milk | orange | | |
| 8 | 90µl | 10µl acridine | | 100µl PBS |
| | milk | orange | | |
| 9 | 99µl | 2µl acridine | | |
| | milk | orange | | |
| 10 | 50µl | 50µl acridine | 10µl | 100µl PBS |
| | milk | orange | EDTA | |
| 11 | 90µl | 10µl acridine | 10µl | |
| | milk | orange | EDTA | |
| 12 | 99µl | 2µl acridine | 10µl | 100µl PBS |
| | milk | orange | EDTA | |

After reacting for 1-2 minutes at room temperature, each combination is measured on the standard flow cytometer used in Examples 1 and 2 essentially in the manner described in Examples 1 and 2 above. In the flow cytometer the sample is measured with a speed of 14µl/min and is excited by a blue laser (488nm). A FL1-H value of 400,000 is used as trigger signal. Fluorescent signals (FL1-H, FL2-), and light scatter signals (side scatter, SSC-H) are used.

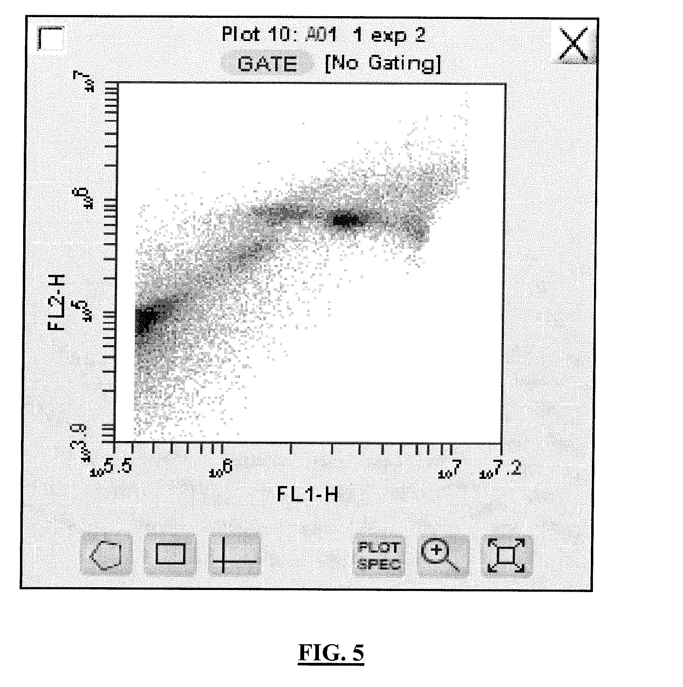

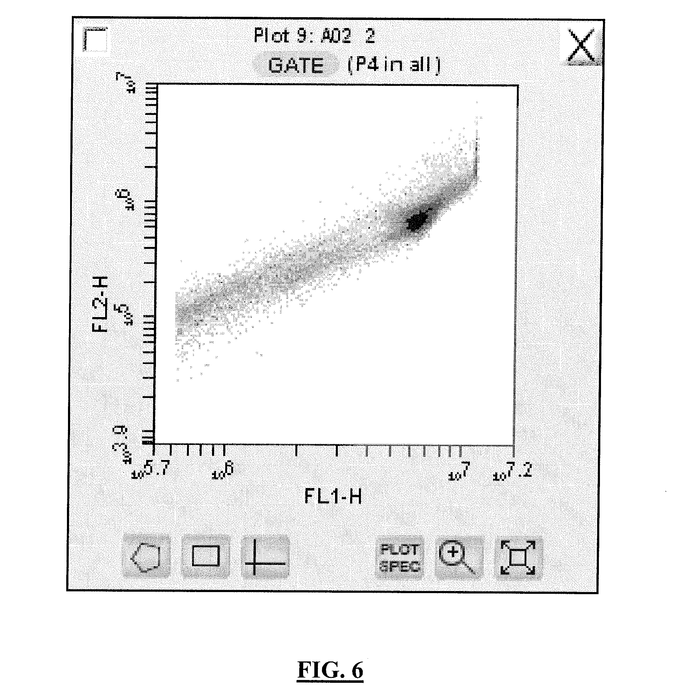

For data analysis, the red vs. green (FL2-H vs FL1-H) fluorescence is plotted and the results are illustrated in Fig.5. When using 2µl or 10µl acridine orange only one cell population is seen in the plots (Fig. 6), i.e. low concentrations of acridine orange do not provide sufficient differentiation. In contrast hereto several populations are seen in plot when using 50µl acridine orange (see Fig. 5.).

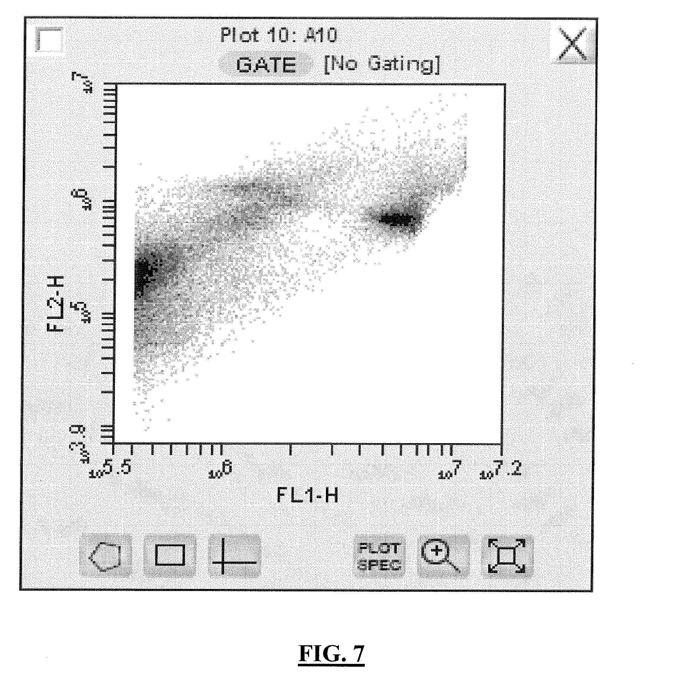

When the high concentration of acridine orange is combined with EDTA the very dense population moves to the right, i.e. the green fluorescence intensities of these cells increase (Fig.7.) as compared to what is illustrated in Fig. 5.

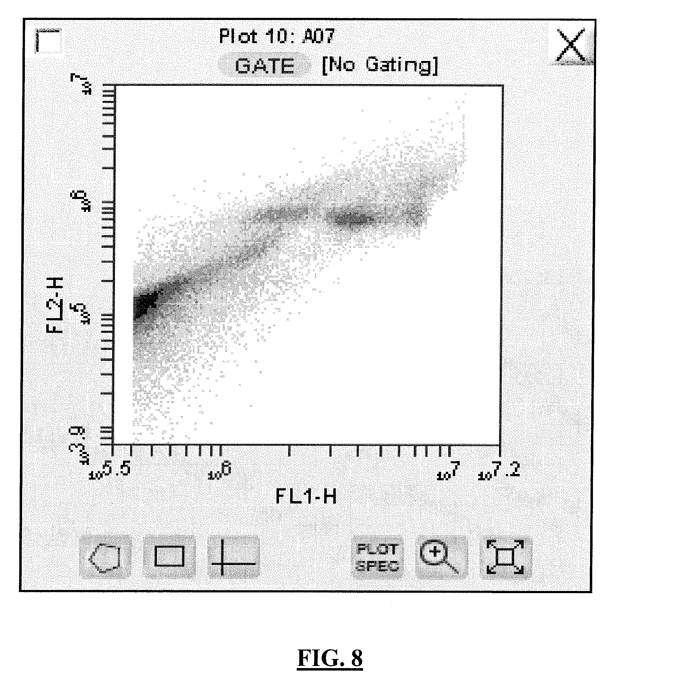

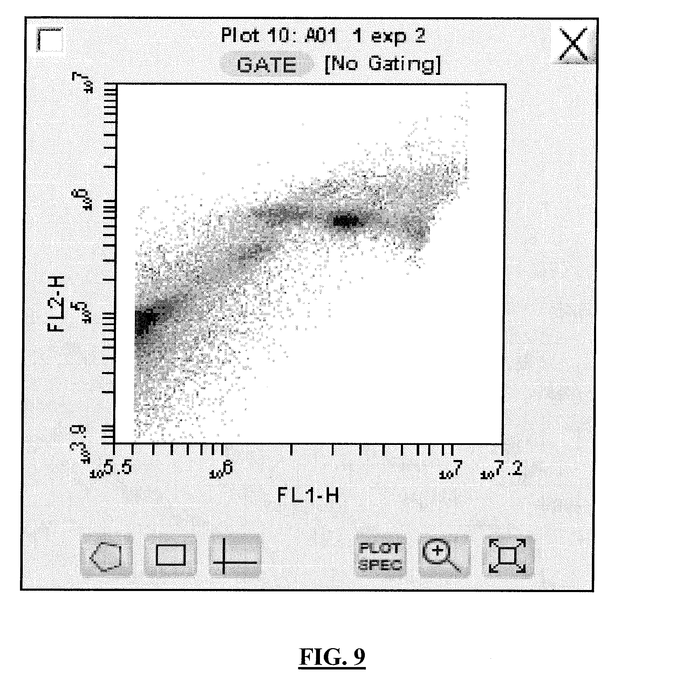

Adding PBS to the sample decreases the noise level, i.e. as shown in Fig. 8 and Fig. 9 it is easier to separate cells from noise. The noise is the wedge shaped population in the left part of the plots. In the plot of Fig. 8 (the sample without PBS) the noise population overlaps one of the cell populations. In the plot of Fig. 9 (the sample with PBS) the noise population is better separated from one of the cell populations.

It can be concluded from this that concentrations of acridine orange greater than around 0.2 mg/ml gives suitable differentiation. The amount of reagent (either as a single reagent or individual reagents added separately) that can be added to the sample without causing unwanted dilution effects can be determined by consideration of an optimum throughput for a system employing the present method in routine analysis. This amount must provide a dilution less than 1:200, preferably less than 1:50 and most preferably less than 1:5.

Although this experimental determination is illustrated with reference to acridine orange it will be appreciated that following a series of measurements the skilled person can reasonably be expected to determine a suitable range of differentiating marker to employ in the method according to the present invention. It will further be appreciated that while the method has been described with regard to flow cytometry measurements of relative fluorescent intensities as a differentiating measurement for cells other differentiating measurements may be employed dependent on the nature of the differentiating marker employed.

## Claims

1. A method for determining a degree of mastitis infection comprising the steps of
i) preparing an un-isolated sample of mammalian milk in which somatic cells are un-isolated from interfering particles in the mammalian milk by adding a reagent comprising a meta-chromatic stain differentiating marker in an amount sufficient to provide a differentiation between cell types in mammalian milk in a dilution ratio of less than 1:200;
ii) measuring a cell count for each of one or more leukocyte types of somatic cell selected from the group lymphocyte, monocyte, macrophage and polymorphonuclear types of leukocyte differentiated by the marker in the sample by means of a flow cytometer having a detection system sensitive to differences in the differentiating marker resulting from the marker becoming differently associated with different cell types in the sample; and
iii) determining an indication of a degree of mastitis infection dependent on the percentage of polymorphonuclear type of leukocyte in the total number of measured cells as a measured cell count by comparing the measured cell count with one or more reference values each being predetermined to be associated with one or more degrees of mastitis infection.

2. A method as claimed in Claim 1 **characterised in that** there is an additional step of measuring a total somatic cell count and **in that** the step of determining an indication further comprises comparing the measured total somatic cell count with one or more reference values each being predetermined to be associated with one or more degrees of mastitis infection.

3. A method as claimed in any preceding claim **characterised in that** the mammalian milk is bovine milk and **in that** determining an indication of a degree of mastitis infection comprises determining an indication of one or both the presence and degree of mastitis.

4. A method as claimed in Claim 3 **characterised in that** the indication of mastitis comprises an indication of sub-clinical mastitis.

5. A method as claimed in Claim 3 or Claim 4 **characterised in that** a degree of mastitis infection indicative of a likelihood of developing clinical mastitis is determined.

6. A method as claimed in Claim 6 **characterised in that** the meta-chromatic stain is acridine orange in a concentration of greater than 0.2 mg/ml.

## Patentansprüche

1. Verfahren zum Bestimmen eines Grads einer Mastitis-Infektion, das die folgenden Schritte umfasst:
i) Vorbereiten einer nicht isolierten Probe von Säugermilch, in der somatische Zellen von störenden Partikeln in der Säugermilch nicht isoliert sind, durch Zugeben eines Reagens, das einen metachromatisch färbenden Differenzierungsmarker in einer Menge, die dazu ausreicht, eine Differenzierung zwischen Zelltypen in Säugermilch bereitzustellen, in einem Verdünnungsverhältnis von weniger als 1:200 umfasst;
ii) Messen einer Zellzahl für jeden eines oder mehrerer Leukozytentypen von somatischen Zellen, die aus der Gruppe von Lymphozyten, Monozyten, Makrophagen und polymorphonukleären Typen von Leukozyten ausgewählt sind und die von dem Marker in der Probe differenziert wurden, mittels eines Durchflusszytometers mit einem Nachweissystem, das für Unterschiede in dem Differenzierungsmarker empfindlich ist, die daraus resultieren, dass der Marker mit unterschiedlichen Zelltypen in der Probe unterschiedlich assoziiert wird; und
iii) Bestimmen einer Anzeige eines Grads einer Mastitis-Infektion in Abhängigkeit von dem Prozentanteil eines polymorphonukleären Typs von Leuktozyten an der Gesamtzahl der gemessenen Zellen als eine gemessene Zellzahl durch Vergleichen der gemessenen Zellzahl mit einem oder mehreren Referenzwerten, von denen jeweils vorherbestimmt wurde, dass sie mit einem oder mehreren Graden einer Mastitis-Infektion assoziiert sind.

2. Verfahren nach Anspruch 1, **dadurch gekennzeichnet, dass** es einen zusätzlichen Schritt des Messens einer Gesamtzahl somatischer Zellen gibt und dass der Schritt des Bestimmens einer Anzeige weiterhin das Vergleichen der gemessenen Gesamtzahl somatischer Zellen mit einem oder mehreren Referenzwerten umfasst, von denen jeweils vorherbestimmt wurde, dass sie mit einem oder mehreren Graden einer Mastitis-Infektion assoziiert sind.

3. Verfahren nach einem vorhergehenden Anspruch, **dadurch gekennzeichnet, dass** die Säugermilch Kuhmilch ist und dass das Bestimmen einer Anzeige eines Grads einer Mastitis-Infektion das Bestimmen einer Anzeige eines oder beider des Vorliegens und des Grads einer Mastitis umfasst.

4. Verfahren nach Anspruch 3, **dadurch gekennzeichnet, dass** die Anzeige einer Mastitis eine Anzeige einer subklinischen Mastitis umfasst.

5. Verfahren nach Anspruch 3 oder 4, **dadurch gekennzeichnet, dass** ein Grad einer Mastitis-Infektion, der eine Wahrscheinlichkeit der Entwicklung einer klinischen Mastitis anzeigt, bestimmt wird.

6. Verfahren nach Anspruch 6, **dadurch gekennzeichnet, dass** die metachromatische Färbung Akridinorange in einer Konzentration von mehr als 0,2 mg/ml ist.

## Revendications

1. - Procédé de détermination d'un degré d'infection mastite comprenant les étapes consistant à :
i) préparer un échantillon non isolé de lait de mammifère dans lequel des cellules somatiques sont non isolées à partir de particules interférentes dans le lait de mammifère par addition d'un réactif comprenant un marqueur de différenciation de colorant métachromatique dans une quantité suffisante pour fournir une différenciation entre des types de cellules dans le lait de mammifère dans un rapport de dilution inférieur à 1:200 ;
ii) mesurer une numérotation cellulaire pour chacun du ou des types de leucocytes de cellule somatique choisis dans le groupe de types de leucocyte : lymphocyte, monocyte, macrophage et polymorphonucléaire, différenciés par le marqueur dans l'échantillon au moyen d'un cytomètre en flux ayant un système de détection sensible aux différences dans le marqueur de différenciation provenant du fait que le marqueur devient associé de façon différente avec les différents types de cellules dans l'échantillon ; et
iii) déterminer une indication d'un degré d'infection mastite dépendant du pourcentage de type polymorphonucléaire de leucocyte dans le nombre total des cellules mesurées comme numérotation cellulaire mesurée par comparaison de la numérotation cellulaire mesurée avec une ou plusieurs valeurs de référence, chacune étant prédéterminée comme étant associée à un ou plusieurs degrés d'infection mastite.

2. - Procédé selon la revendication 1, **caractérisé par le fait qu'**il y a une étape supplémentaire de mesure d'une numérotation de cellules somatiques totales et **par le fait que** l'étape de détermination d'une indication comprend en outre la comparaison de la numérotation de cellules somatiques totales mesurée avec une ou plusieurs valeurs de référence, chacune étant prédéterminée comme étant associée à un ou plusieurs degrés d'infection mastite.

3. - Procédé selon l'une quelconque des revendications précédentes, **caractérisé par le fait que** le lait de mammifère est du lait de bovin et **par le fait que** la détermination d'une indication d'un degré d'infection mastite comprend la détermination d'une indication de l'un ou des deux de la présence et du degré de mastite.

4. - Procédé selon la revendication 3, **caractérisé par le fait que** l'indication de mastite comprend une indication de mastite sub-clinique.

5. - Procédé selon l'une des revendications 3 ou 4, **caractérisé par le fait qu'**un degré d'infection mastite indicateur d'une probabilité de développer une mastite clinique est déterminé.

6. - Procédé selon la revendication 6, **caractérisé par le fait que** le colorant métachromatique est l'acridine orange dans une concentration supérieure à 0,2 mg/ml.
